(19) Europäisches Patentamt
European Patent Office
Office européen des brevets

(11) Veröffentlichungsnummer: **0 198 232 B1**

(12) **EUROPÄISCHE PATENTSCHRIFT**

(45) Veröffentlichungstag der Patentschrift: **29.05.91**

(51) Int. Cl.[5]: **C07C 1/26**, C07C 17/00, C07B 35/06, C07C 45/41, C07D 307/88, C07D 213/48

(21) Anmeldenummer: **86103448.6**

(22) Anmeldetag: **14.03.86**

Die Akte enthält technische Angaben, die nach dem Eingang der Anmeldung eingereicht wurden und die nicht in dieser Patentschrift enthalten sind.

# (54) Verfahren zur reduktiven Dehalogenierung von Halogenverbindungen.

(30) Priorität: **20.03.85 DE 3510033**

(43) Veröffentlichungstag der Anmeldung:
**22.10.86 Patentblatt 86/43**

(45) Bekanntmachung des Hinweises auf die Patenterteilung:
**29.05.91 Patentblatt 91/22**

(84) Benannte Vertragsstaaten:
**AT BE CH DE FR GB IT LI NL**

(56) Entgegenhaltungen:
**BE-A- 525 596**
**DE-B- 1 057 080**
**US-A- 3 260 761**
**US-A- 4 155 941**

(73) Patentinhaber: **BASF Aktiengesellschaft**
**Carl-Bosch-Strasse 38**
**W-6700 Ludwigshafen(DE)**

(72) Erfinder: **Dockner, Toni, Dr.**
**Grossgasse 6**
**W-6701 Meckenheim(DE)**
Erfinder: **Krug, Herbert**
**Mussbacher Strasse 49**
**W-6700 Ludwigshafen(DE)**
Erfinder: **Nohe, Heinz, Dr.**
**Ruppertsberger Strasse 11**
**W-6701 Meckenheim(DE)**

**EP 0 198 232 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die reduktive Dehalogenierung von vicinalen Dihalogeniden.

Es ist bekannt, die reduzierende Eliminierung von vicinalen Dihalogeniden mit Metallen wie Na. K, Mg, Zn, Cu mit Organometallverbindungen oder mit Metallhydriden durchzuführen. Eine Zusammenfassung dieser Methoden findet sich in Houben-Weyl, Methoden der Organischen Chemie, 4. Aufl., Bd. V/1b, 1972.

Die hydrierende Dehalogenierung aliphatischer Halogenverbindungen mit Raney-Nickel in Gegenwart einer zum Halogen äquivalenten Menge an Alkali ist in Chem.Ber. 92. 1700 (1959) beschrieben worden. R. Baltzly und A.P. Phillips lehren die katalytische Hydrogenolyse von aromatischen Halogenverbindungen (J.Amer.Chem.Soc. 68, 261-265, 1946) in neutraler oder saurer wäßriger oder alkoholischer Lösung, wobei als Katalysator Edelmetalle wie Palladium oder Platin, z.B. Palladium/Tierkohle oder Adams-Katalysator Verwendung finden. Nach G.E. Ham und W.P. Coker lassen sich in Vinyl- oder Allylstellung halogensubstituierte Olefine in Gegenwart von Rhodium, Palladium oder Platin zu Halogenalkanen hydrieren. Die Ausbeuten sind abhängig vom Lösungsmittel, Katalysatorträger und Vorhandensein von Thiophen (J.Org.Chem. 29. 194-198, 1964).

Die Entfernung aromatisch gebundenen Halogens und Ersatz durch Wasserstoff gelingt ebenfalls durch Metalle wie Na, K oder metallorganische Verbindungen oder auch Hydride (siehe Houben-Weyl, Methoden d. org. Chem. , Bd. 5/4, S. 769, 1960). Darüber hinaus läßt sich Halogen in der aromatischen Reihe durch katalytisch angeregten Wasserstoff ersetzen. Als Katalysatoren werden Palladium und Raney-Nickel vorgeschlagen.

Die selektive Dehalogenierug von Acylchloriden zur Herstellung von Aldehyden ist in Houben-Weyl, Methoden der organischen Chemie, Band VII/1, S. 285ff und Bd. 4/1c, S. 370, beschrieben. Dazu wird in der Regel die Rosenmund-Reduktion (Merck-Index 10. Aufl. ONR-78) angewandt, nach der Acylchloride in Gegenwart eines Katalysators wie Palladium auf Bariumsulfat hydriert werden. Häufig ist es nötig, den Katalysator zu inaktivieren, um eine Weiterreduktion zum Alkohol zu unterdrücken, indem man Katalysatorgifte z.B. "Chinolin-Schwefel" oder Thioharnstoff zusetzt. Eine Schwefelüberdosierung oder die Anwesenheit von Phosphorverbindungen bewirken ein Ausbleiben der Enthalogenierung. Wesentlich für das Gelingen dieses Verfahrens ist weiterhin die Verwendung völlig trockener Lösungsmittel, die frei von unkontrollierbaren Katalysatorgiften sein müssen.

Nachteilig an den beschriebenen Verfahren zur reduktiven Dehalogenierung ist, daß sie technisch nicht oder nur eingeschränkt zu nutzen sind, da die Reagenzien sehr teuer sind und große Salzmengen, in vielen Fällen Schwermetallsalze anfallen.

In der japanischen Offenlegungsschrift 216 ,075/1983 wird ein Verfahren zur Enthalogenierung von polychloriertem Biphenyl (PCB) beschrieben, wonach PCB oder Stoffe, die PCB enthalten, unter Sauerstoffausschluß in der Gasphase bei hohen Temperaturen von 400 bis 800 °C mit Kohlenstoff in Kontakt gebracht werden. Beispielsweise können so geringe Mengen (1000 ppm) an PCB, gelöst in Hexan, verdampft und an Aktivkohle (5 g) zersetzt werden.

Aus der US-A- 3 260 761 ist ein Verfahren bekannt, das perhalogenierte Alkane in perhalogenierte Alkene überführt, wobei das als Lösungsmittel fungierende Alkan zum Alkylhalogenid halogeniert wird und nur ein Teil des Halogenids als Säure HX anfällt.

Es bestand nun die Aufgabe, ein Verfahren zu finden, das sich technisch leicht durchführen läßt, auch im großtechnischen Maßstab anwendbar ist und die beschriebenen Nachteile überwindet.

Demgemäß wurde ein Verfahren zur reduktiven Dehalogenierung vicinaler Dihalogenide der allgemeinen Formel I

$$\begin{array}{ccccccc} R & - & CH & - & CH & - & R' \\ & & | & & | & & \\ & & X & & X & & \end{array} \qquad (I),$$

in der X für Iod, Brom und/oder Chlor steht und die Reste R und R' unabhängig voneinander Wasserstoff, eine Alkylgruppe, eine Cycloalkylgruppe, eine Arylgruppe oder eine Aralkylgruppe bedeuten oder beide Reste zusammen mit den C-Atomen, an die sie gebunden sind, zu einem Cycloalkan mit 4 bis 12 Kohlenstoffatomen verbunden sind, welches dadurch gekennzeichnet ist, daß man die Halogenverbindungen in der Flüssigphase mit Alkanen unter zusatz von elementarem Kohlenstoff bei Temperaturen von 100 bis 450 °C unter Bildung von Alkenen und Halogenwasserstoffen umgesetzt.

Die reduktive Eliminierung vicinaler Dihalogenide wird durch folgende Gleichung beschrieben:

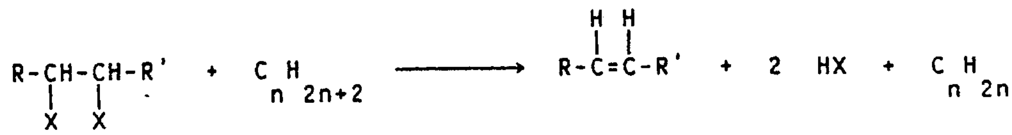

R, R' = H, Alkyl, Cycloalkyl, Aryl, Aralkyl X = Cl, Br, I

Der für die Bildung von Halogenwasserstoff benötigte Wasserstoff entstammt dem Alkan, aus dem neben wasserstoffärmeren Derivaten überwiegend Kohlenstoff gebildet wird.

Als Alkane werden zweckmäßig hochsiedende Mineralöle verwendet, deren Siedetemperaturen höher liegen als die Reaktionstemperatur, die 100 bis 450, vorzugsweise 200 bis 400, insbesondere 250 bis 350 °C beträgt. Solche Kohlenwasserstoffe sind z.B. Vakuum-Gasöl, technisches Weißöl, Heizöl S, Vakuumrückstandsöl oder sonstige bei der Erdölfraktionierung anfallende hochsiedende Bestandteile. Es ist auch möglich, niedrigersiedende Kohlenwasserstoffe wie Heizöl L, Benzin, Leichtbenzin oder gar Flüssiggas zu verwenden (siehe dazu Ullmann, Enzyklopädie d. techn. Chemie, 3. Aufl., Bd. 6, S. 595 bis 760, 1955 und 4. Aufl., Bd. 12, S. 570 bis 573). Um die Reaktion in der Flüssigphase durchführen zu können, sind dann jedoch erhöhte Drucke erforderlich. Rohöle verschiedener Herkunft sind ebenso einsetzbar.

Die Alkane werden vorteilhaft in einer Menge von 10 bis 1000 g, insbesondere 20 bis 80 g pro Mol Halogenverbindung verwendet. Zur Reduktion von Acylhalogeniden haben sich Mengen von 10 bis 2000 g, insbesondere 100 bis 1000 g pro Mol Ausgangsstoff bewährt.

Für die erfindungsgemäße Umsetzung in Betracht kommende Ausgangsstoffe sind vicinale Dihalogenide der allgemeinen Formel I

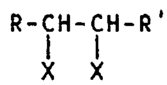 I,

in der X für Iod, Brom und/oder Chlor steht und die Reste R und R' unabhängig voneinander Wasserstoff, einen aliphatischen Rest, z.B. eine Alkylgruppe mit 1 bis 20, insbesondere 1 bis 5 Kohlenstoffatomen, eine Cycloalkylgruppe, z.B. mit 5 bis 8 Kohlenstoffatomen, eine Arylgruppe oder eine Aralkylgruppe, insbesondere mit 6 bis 12 Kohlenstoffatomen bedeuten oder beide Reste zusammen mit den C-Atomen an die sie gebunden sind zu einem Cycloalkan mit 4 bis 12, insbesondere 4 bis 8 Kohlenstoffatomen verbunden sind.

Beispielsweise kommen Verbindungen wie 1 ,2-Dichlorpropan, 1 ,2-Dichlorbutan, 1 ,2-Dichlorpentan, 1 ,2-Dibrombutan, 2,3-Diiodbutan, 1 ,2-Dibrom-2-methylpropan, 1 ,2-Dibromcyclobutan oder -cyclohexan, 1 ,2-Dichlor-1,2-diphenylethan, 1,2-Dichlor-3-phenyl-propan, 1-Brom-2-iodbutan, 1-Brom-2-chlorethan in Betracht,

Durch die beschriebenen Ausgangsverbindungen soll die Anwendungsbreite des erfindungsgemäßen Verfahrens nicht eingeschränkt werden. Die Halogenverbindungen können zusätzlich unter den Reaktionsbedingungen inerte Substituenten wie Cyano-, Alkoxy-, Dialkylamino-, Phenyl- oder substituierte Phenylgruppen tragen. Weiterhin können heterocyclische Verbindungen, z.B. halogensubstituiertes Pyridin, Chinolin, Pyrrol oder Imidazol umgesetzt werden.

Die Reaktion wird in flüssiger Phase durchgeführt, wobei mindestens der Hauptteil der reagierenden Kohlenwasserstoffe flüssig vorliegen sollte. Die flüssige Phase enthält suspendierten Kohlenstoff, der entweder als Bestandteil der eingesetzten Alkane vorhanden ist oder gesondert zugesetzt wird. Vorzugsweise liegen im Reaktionsgemisch 1 bis 50, insbesondere 5 bis 20 Gew.% Kohlenstoff, bezogen auf das verwendete Alkan vor. Geeignete Kohlenstoffzusätze sind z.B. Petrolkoks und Ruß oder eine andere Form das Graphits, besonders vorteilhaft verwendet man Aktivkohlen wie Carboraffin P® oder Tierkohle, die z.B. mit $ZnCl_2$, Phosphorsäure oder Wasserstoff aktiviert sind.

Die erfindungsgemäße Umsetzung kann diskontinuierlich oder kontinuierlich bei Normaldruck, erhöhtem oder vermindertem Druck nach den dafür üblichen Techniken, z.B. in einem Rührreaktor oder in einem zylindrischen Reaktor mit Umlauf durchgeführt werden.

Die Dehalogenierung wird zweckmäßigerweise so durchgeführt, daß man wie der Abbildung zu entnehmen ist, die Halogenverbindungen (1) fest, flüssig oder gasförmig, gegebenenfalls zusammen mit einem Inertgas (2), z.B. Stickstoff dem Reaktor (3) mit der auf Reaktionstemperatur erhitzten flüssigen elementaren Kohlenstoff enthaltenden Alkanphase zuführt. Nach der Umsetzung verlassen die Reaktionsprodukte in der Regel gasförmig zusammen mit dem gebildeten Halogenwasserstoff den Reaktor. Die

Produkte werden isoliert, indem man sie z.B. je nach Siedepunkt entweder vor oder nach Abtrennung des Halogenwasserstoffs kondensiert und gegebenenfalls z.B. destillativ reinigt. Die Abtrennung des Halogenwasserstoffs erfolgt zweckmäßig durch eine Wasserwäsche. Die so erhaltenen Halogenwasserstoffsäuren werden dann neutralisiert oder einer Nutzung zugeführt. Gegebenenfalls kann auch der Halogenwasserstoff unmittelbar durch eine Laugewäsche neutralisiert werden.

Nach dem erfindungsgemäßen Verfahren ist es überraschend auf billige und technisch einfache Art möglich, die Umwelt belastende toxische halogenierte Kohlenwasserstoffe in weniger giftige bzw. unbedenkliche Verbindungen zu überführen, wodurch Entsorgungsprobleme dieser z.T. in großen Mengen bei technischen Prozessen verwendeten bzw. anfallenden Stoffe gelöst werden, ohne daß die bei der Verbrennung üblichen Probleme auftreten. Über die Toxikologie halogenierter Kohlenwasserstoffe, insbesondere Chlorkohlenwasserstoffe siehe Ullmann's Enzyklopädie der techn. Chemie, 4. Aufl., Bd. 9, Seiten 481 bis 490, 521, 522 und 536.

## Beispiel 1

Wie der abgebildeten Apparatur zu entnehmen ist, wurden durch einen 2 l-Rührkolben (3), gefüllt mit 1,2 kg als Vakuumrückstand bezeichnete hochsiedende Mineralölfraktion bei 350 °C stündlich 40 ml 1 ,2-Dichlorpropan (1) und 8 l Stickstoff (2) geleitet.

Um die Reaktionstemperatur halten zu können, wurde nicht umgesetztes Dichlorpropan zusammen mit Leichtsiedern durch Kondensation (4) bei 90 °C aus dem Reaktionsgemisch entfernt.

Pro Stunde fielen 12,75 g Kondensat (5) mit 80 Gew.% Dichlorpropan an. Daraus errechnet sich ein Umsatz von 78 %. Die gasförmigen Reaktionsprodukteleitete man zwecks Entfernung der Salzsäure durch eine Waschkolonne (6). Man erhielt stündlich 15 l Abgas (7) mit 43 Vol.% Propen. Dies entspricht einer Ausbeute von 90 %, bezogen auf den Umsatz. Stündlich fielen in der Waschkolonne 217 g wäßrige HCl mit 10,5 Gew.% HCl an. Dies entspricht 76 % des im durchgesetzten Dichlorpropan enthaltenen Cl und entspricht dem Umsatz.

## Beispiel 2

Man verfuhr wie in Beispiel 1, setzte aber 1,2 kg als technisches Weißöl bezeichnete paraffinische Kohlenwasserstoff-Fraktion ein. Pro Stunde fielen 49 g Kondensat mit 77 Gew.% Dichlorpropan an, dies entspricht einem Umsatz von 17 %.

An Salzsäure wurden 210 g 2,43 Gew.% gewonnen. Dies entspricht 17 % des eingesetzten Cl. Man erhielt 9 l Abgas mit 8,9 Vol.% $C_3H_6$ Dies entspricht einer Ausbeute von 51 %. bezogen auf den Umsatz.

## Beispiel 3

Man verfuhr wie in Beispiel 1, setzte aber 0,75 kg technisches Weißöl mit 0,25 kg Carboraffin P® ein. Pro Stunde fielen 14,4 g Kondensat mit 64 Gew.% Dichlorpropan an. Dies entspricht einem Umsatz von 80 %. Daneben erhielt man 225 g wäßrige HCl mit 10,5 Gew.% HCl. Dies entspricht 79 % des eingesetzten Cl. Man erhält 16 l Abgas mit 41,3 Vol.% Propen. Dies entspricht einer Ausbeute von 90 %. bezogen auf den Umsatz.

## Beispiel 4

Man verfuhr wie in Beispiel 1, setzte aber 40 ml 1 ,2-Dibrombutan mit 4,5 l Stickstoff sein. Man erhielt stündlich 14,5 g Kondensat mit 76 Gew.% Dibrombutan. Dies entspricht einem Umsatz von 85 %. Es fielen 385 g wäßrige HBr mit 12 Gew.% HBr und 8 l Abgas mit 40 Vol.% Buten-1 an. Dies entspricht 85 % des eingesetzten Brom und einer Ausbeute an Buten-1 von 50 %, bezogen auf den Umsatz.

## Beispiel 5

Man verfuhr wie in Beispiel 3, wählte aber als Reaktionstemperatur 250 °C. Pro Stunde fielen 49 g

Kondensat mit 74 Gew.% Dichlorpropan an. Dies entspricht einem Umsatz von 21 Gew.%. Man erhält 9 l Abgas mit 10,2 Vol.% Propen. Dies entspricht einer Ausbeute von 48 %` bezogen auf umgesetztes Dichlorpropan.

Beispiel 6

Man verfuhr wie in Beispiel 1, setzte aber 1,2 kg als Vakuumgasöl bezeichnete hochsiedende Mineralölfraktion ein. Pro Stunde fielen 39 g Kondensat mit 85,6 Gew.% Dichlorpropan an. Dies entspricht einem Umsatz von 28 %. Man erhielt daneben 10,5 l Abgas mit 22 Vol.% Propen, dies entspricht einer Ausbeute von 92 %, bezogen auf umgesetztes Dichlorpropan.

## Ansprüche

1. Verfahren zur reduktiven Dehalogenierung vicinaler Dihalogenide der allgemeinen Formel I

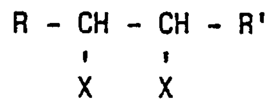

(I),

in der X für Iod, Brom und/oder Chlor steht und die Reste R und R' unabhängig voneinander Wasserstoff, eine Alkylgruppe, eine Cycloalkylgruppe, eine Arylgruppe oder eine Arylkylgruppe bedeuten oder beide Reste zusammen mit den C-Atomen, an die sie gebunden sind, zu einem Cycloalkan mit 4 bis 12 Kohlenstoffatomen verbunden sind, dadurch gekennzeichnet, daß man die Halogenverbindungen in der Flüssigphase mit Alkanen unter zusatz von elementarem Kohlenstoff bei Temperaturen von 100 bis 450 ° C unter Bildung von Alkenen und Halogenwasserstoffen umsetzt.

2. Verfahren gemäß Anspruch 1 , dadurch gekennzeichnet, daß man als Alkane hochsiedende Mineralöle verwendet, deren Siedepunkte höher liegen als die Reaktionstemperatur.

3. Verfahren gemäß Anspruch 1 , dadurch gekennzeichnet, daß man als Alkane Vakuumrückstand, Heizöl S oder technisches Weißöl verwendet.

4. Verfahren gemäß Anspruch 1 , dadurch gekennzeichnet, daß im Reaktionsgemisch 1 bis 50 Gew.% Kohlenstoff, bezogen auf den Kohlenwasserstoff vorliegt.

5. Verfahren gemäß Anspruch 1, dadurch gekennzeichnet, daß man als Alkane niedrigsiedende Kohlenwasserstoffe wie Heizöl L, Benzin, Leichtbenzin oder Flüssiggase verwendet und die Umsetzung unter erhöhtem Druck durchführt.

## Claims

1. A process for reductively dehalogenating a vicinal dihalide of the formula I

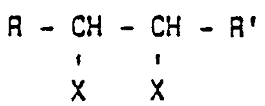

(I)

where X is iodine, bromine or chlorine and R and $R^1$ are each independently of the other hydrogen, alkyl, cycloalkyl, aryl or arylkyl or are, together with the carbon atoms to which they are bonded, a cycloalkane of 4 to 12 carbon atoms, which comprises reacting the halogen compound in the liquid

phase with an alkane in the presence of elemental carbon at from 100 to 450 °C with the formation of an alkene and a hydrogen halide.

2. A process as claimed in claim 1, wherein the alkanes used are high-boiling mineral oils whose boiling points are higher than the reaction temperature.

3. A process as claimed in claim 1, wherein the alkanes used are vacuum residue, heavy fuel oil or technical grade white oil.

4. A process as claimed in claim 1, wherein the reaction mixture contains from 1 to 50% by weight of carbon, based on the hydrocarbon.

5. A process as claimed in claim 1, wherein the alkanes used are low-boiling hydrocarbons such as light fuel oil, gasoline, naphtha or liquid gases and the reaction is carried out under superatmospheric pressure.

## Revendications

1. Procédé de déshalogénation par voie de réduction de dihalogénures vicinaux de formule générale I

$$R - \underset{X}{CH} - \underset{X}{CH} - R' \qquad (I)$$

dans laquelle X est mil pour un atome d'iode, de brome et/ou de chlore et les restes R et R' représentent chacun, indépendamment l'un de l'autre, un atome d'hydrogène, un groupement alkyle, un groupement cycloalkyle, un groupement aryle ou un groupement arylalkyle ou les deux restes forment ensemble, avec les atomes de carbone auxquels ils sont fixés, un cycloalcane à 4-12 atomes de carbone, caractérisé en ce qu'on fait réagir les composés halogénés en phase liquide avec des alcanes, avec addition de carbone élémentaire, à des températures de 100 à 450 °C, avec formation d'alcènes et d'hydrocarbures halogénés.

2. Procédé selon la revendication 1, caractérisé en ce qu'an utilise, comme alcane, des huiles minérales de point d'ébullition élevé, dont les points d'ébullition se situent au-dessus de la température de réaction.

3. Procédé selon la revendication 1, caractérisé en ce qu'on utilise, comme alcane, un résidu court de la distillation poussée, une huile lourde ou une huile blanche technique.

4. Procédé selon la revendication 1, caractérisé en ce qu'on introduit au préalable, dans le mélange réactionnel, de 1 à 50% en poids de carbone par rapport à l'alcane.

5. Procédé selon la revendication 1, caractérisé en ce qu'on utilise, comme alcane, des hydrocarbures de bas point d'ébullition tels que du fioul L, de l'essence ordinaire, de l'essence légère ou des gaz liquéfiés et on conduit la réaction sous pression élevée.

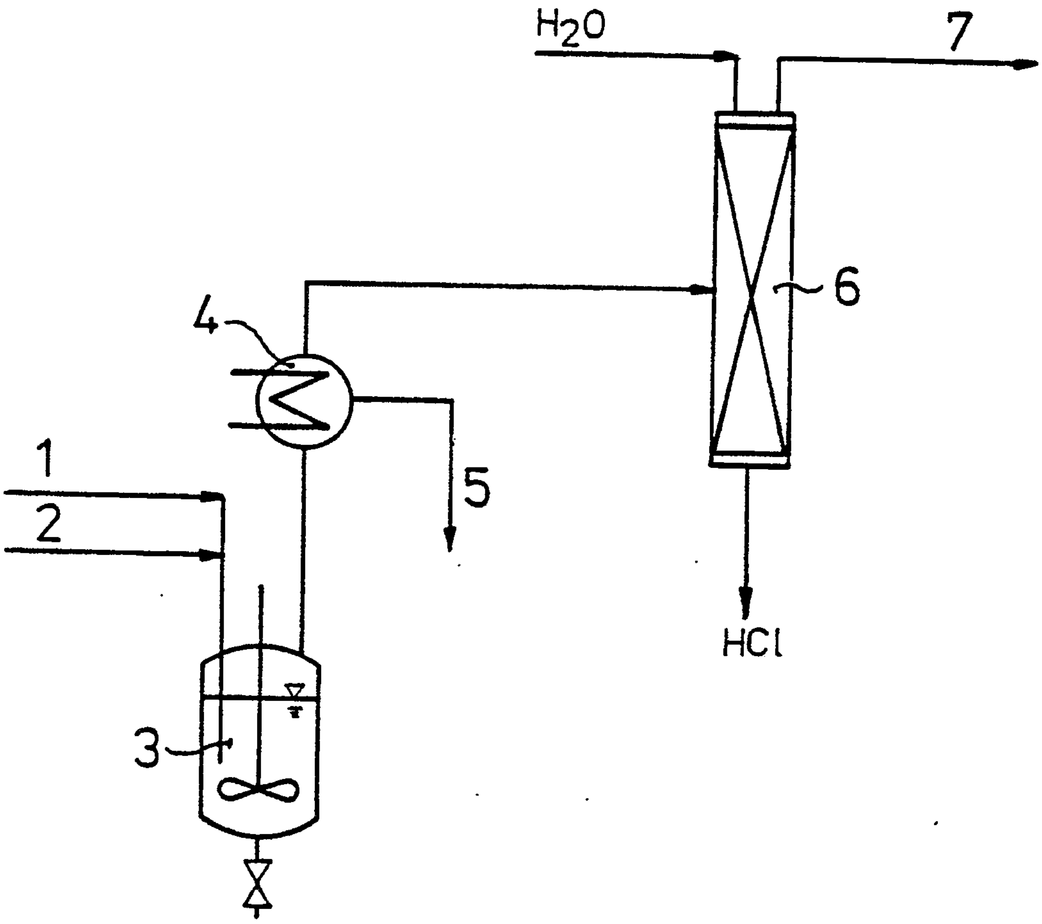
H2O
7
6
4
1
2
5
HCl
3